Europäisches Patentamt

(19) European Patent Office

Office européen des brevets

(11) Publication number: **0 013 474**
**B1**

(12) **EUROPEAN PATENT SPECIFICATION**

(45) Date of publication of patent specification: **29.06.83**

(21) Application number: **79302493.6**

(22) Date of filing: **07.11.79**

(51) Int. Cl.³: **C 07 D 213/61**

(54) **Preparation of 2-chloro-5-trifluoromethylpyridine.**

(30) Priority: **07.12.78 GB 4758378**
**22.01.79 GB 7902196**

(43) Date of publication of application:
**23.07.80 Bulletin 80/15**

(45) Publication of the grant of the patent:
**29.06.83 Bulletin 83/26**

(84) Designated Contracting States:
**AT BE CH DE FR GB IT LU NL SE**

(56) References cited:
**EP - A - 0 001 473**
**US - A - 4 054 499**

(73) Proprietor: **IMPERIAL CHEMICAL INDUSTRIES PLC**
**Imperial Chemical House Millbank**
**London SW1P 3JF (GB)**

(72) Inventor: **Roberts, Norman Leyland**
**7 Cranleigh Close**
**Walton Nr. Warrington, Cheshire (GB)**
Inventor: **Whittaker, Graham**
**3 Buttermere Close**
**Frodsham, Cheshire (GB)**
Inventor: **O'Brien, Anne**
**26 Kingsley Road**
**Frodsham, Cheshire (GB)**

(74) Representative: **Roberts, John Stanley et al,**
**IMPERIAL CHEMICAL INDUSTRIES PLC Legal**
**Department: Patents Thames House North**
**Millbank**
**London SW1P 4QG (GB)**

Courier Press, Leamington Spa, England

## Preparation of 2-chloro-5-trifluoromethylpyridine

This invention relates to the preparation of 2-chloro-5-trifluoromethylpyridine.

2-chloro-5-trifluoromethylpyridine is a desirable intermediate for use in the preparation of compounds having herbicidal activity, for example in the preparation of herbicidal pyridine compounds described in EP—A—1473.

In GB 1 599 783; and US—A—4 205 175 there is described a method of partial chlorination of 3-methylpyridine to give products containing a single chlorine atom in the pyridine ring and either two or three chlorine atoms as substituents in the methyl group. Among the products of the process there described is 2-chloro-5-trichloromethylpyridine, which may subsequently be fluorinated to yield 2-chloro-5-trifluoromethylpyridine or 2-chloro-5-perchlorofluoromethylpyridines (for example 2-chloro-5-chlorodifluoromethylpyridine).

In the partial chlorination of 3-methylpyridine the desired 2-chloro-5-trichloromethylpyridine is, however, accompanied by substantial proportions of other partially-chlorinated derivatives which it can be difficult to separate from the desired product; this may lead to waste of material in the route to 2-chloro-5-trifluoromethylpyridine from 3-methylpyridine via 2-chloro-5-trichloromethylpyridine. Earlier prior art, McBee et al. Ind. and Eng. Chem. 39 (1947) 389—391 relating to the chlorination of methylpyridines in the liquid phase stated that 3-methylpyridine "could not be chlorinated to any identifiable products".

We have now found that 2-chloro-5-trifluoromethylpyridine may selectively be prepared by chlorination of 3-trifluoromethylpyridine. Furthermore, the separation of the desired product from the by-products may usually be achieved more readily than separation of 2-chloro-5-trichloromethylpyridine from the by-products obtained in the chlorination of 3-methylpyridine.

According to the present invention there is provided a process for the preparation of 2-chloro-5-trifluoromethylpyridine characterised in that 3-trifluoromethylpyridine is chlorinated:—

(i) in the vapour phase optionally in the presence of a diluent at a temperature in the range from 100°C to 500°C, provided that when the temperature is below 250°C the chlorination is carried out in the presence of ultraviolet radiation, or

(ii) in the liquid phase in an organic solvent in the presence of ultraviolet radiation and/or in the presence of a free-radical initiator.

When the chlorination is carried out in the vapour phase it is preferred to use at least 1 mole of chlorine per mole of 3-trifluoromethylpyridine. The vapour phase chlorination process is preferably carried out at a temperature in the range from 300°C to 450°C. The preferred proportion of chlorine will depend upon the reaction temperature. In general in the vapour-phase chlorination, it is preferred to use at least 1 mole of chlorine (for example from 1 to 6 moles) per mole of 3-trifluoromethylpyridine but at relatively high temperatures, for example at temperatures above 400°C, the use of more than about 2 moles of chlorine per mole of 3-trifluoromethylpyridine may increase the proportion of products containing two or more chlorine atoms as substituents in the pyridine ring.

The vapour-phase chlorination is preferably carried out in the presence of a diluent; this may be inorganic, for example nitrogen and/or steam, but is preferably organic. When an organic diluent is used, this is preferably a compound which is inert towards chlorine (for example carbon tetrachloride, which is the diluent especially preferred) or a compound such that any reaction with chlorine yields a product which is inert to further chlorination (for example chloroform, which may yield carbon tetrachloride).

When a gaseous diluent is used the 3-trifluoromethylpyridine starting material may be vapourised in the stream of diluent vapour which serves as a carrier gas; when a liquid diluent is used, the starting material may be dissolved in the liquid diluent and the resulting solution may then be vaporised as a whole.

In the vapour-phase chlorination, convenient residence times of the mixture in the reaction zone are, for example, between 10 and 30 seconds, but higher or lower residence times may be used. The liquid-phase chlorination process is carried out in the presence of an organic diluent of the kind already described in respect of the vapour-phase process; again, carbon tetrachloride is a very convenient diluent.

The liquid-phase chlorination may be carried out over a wide range of temperature, depending partly upon the solvent employed. In general, the reaction is conveniently carried out at a temperature in the range from 0°C to 100°C, especially from 20°C to 80°C, and conveniently under conditions of reflux. Superatmospheric pressure may be used if desired or if necessary in order to maintain the reaction mixture in the liquid phase. Suitable free-radical initiators include peroxides (for example dibenzoyl peroxide; di-tertiary-butyl peroxide), azonitriles (for example alpha, alpha-azobisisobutyronitrile) and halides of transition metals.

It will be understood that under some circumstances, for example when reflux conditions are employed, the desired chlorination process may occur both in the liquid phase and in the vapour phase.

The desired 2-chloro-5-trifluoromethylpyridine may be recovered from the reaction products by methods conventional in the art, for example fractional distillation and fractional crystallization.

The invention is illustrated by the following Examples.

## Example 1

A solution of 3-trifluoromethylpyridine in carbon tetrachloride was fed to a packed vaporiser maintained at a temperature of 300°C. The issuing vapours were passed to a vertical glass tubular reactor of 10 cm bore held at a temperature of 380°C where they were mixed with a stream of chlorine. The residence time was 10.5 seconds.

The initial reaction mixture contained 3.5 moles of chlorine and 48 moles of carbon tetrachloride per mole of 3-trifluoromethylpyridine.

The gaseous reactor effluent over a period of 1 hour was condensed and the condensate was found by gas-liquid chromatography, mass spectrometry and nuclear magnetic resonance, to contain 2-chloro-5-trifluoromethylpyridine as the major product together with 2-chloro-3-trifluoromethylpyridine and 2,6-dichloro-3-trifluoromethylpyridine as by-products. The yields, determined by $^{19}F$ nuclear magnetic resonance using pure 2-fluoro-5-trifluoromethylpyridine as internal standard, were as follows:—

| | |
|---|---|
| 2-chloro-5-trifluoromethylpyridine | 67% |
| 2-chloro-3-trifluoromethylpyridine | 11% |
| 2,6-dichloro-3-trifluoromethylpyridine | 7% |

## Example 2

The procedure of Example 1 was repeated, except that the reaction temperature was 425°C. The yields were as follows:—

| | |
|---|---|
| 2-chloro-5-trifluoromethylpyridine | 56% |
| 2,6-dichloro-3-trifluoromethylpyridine | 25% |
| 2-chloro-3-trifluoromethylpyridine | 4% |

## Example 3

The general procedure was the same as in Example 1. The reaction mixture contained 1.1 mole of chlorine and 48 moles of carbon tetrachloride per mole of 3-trifluoromethylpyridine. The reaction temperature was 425°C and the residence time was 25 seconds.

The yields were as follows:—

| | |
|---|---|
| 2-chloro-5-trifluoromethylpyridine | 60% |
| 2,6-dichloro-3-trifluoromethylpyridine | 18% |
| 2-chloro-3-trifluoromethylpyridine | 7% |

## Example 4

The general procedure was the same as in Example 1. The reaction mixture contained 6 moles of chlorine and 48 moles of carbon tetrachloride per mole of 3-trifluoromethylpyridine. The reaction temperature was 350°C and the residence time was 16 seconds.

The yields were as follows:—

| | |
|---|---|
| 2-chloro-5-trifluoromethylpyridine | 51% |
| 2-chloro-3-trifluoromethylpyridine | 8% |
| 2,6-dichloro-3-trifluoromethylpyridine | 3% |
| (unreacted 3-trifluoromethylpyridine 22%) | |

## Example 5

The general procedure was the same as in Example 1. The reaction mixture contained 3.5 moles of chlorine and 48 moles of carbon tetrachloride per mole of 3-trifluoromethylpyridine. The reaction temperature was 400°C and the residence time was 10.5 seconds.

**0013474**

The yields were as follows:—

| | |
|---|---|
| 2-chloro-5-trifluoromethylpyridine | 64% |
| 2-chloro-3-trifluoromethylpyridine | 9% |
| 2,6-dichloro-3-trifluoromethylpyridine | 14% |

### Example 6

3-trifluoromethylpyridine was vapourised in a stream of nitrogen and mixed with a stream of chlorine to form a reaction mixture containing 0.62 mole of chlorine and 1.3 mole of nitrogen per mole of 3-trifluoromethylpyridine. This mixture was passed through a glass tube irradiated with ultra-violet light, the gas temperature being maintained at 110°C. The residence time was approximately 30 seconds.

The product contained 40 parts by weight of 2-chloro-5-trifluoromethylpyridine, 6 parts by weight of 2-chloro-3-trifluoromethylpyridine and 50 parts by weight of unreacted 3-trifluoromethylpyridine. This corresponds to a yield of 62% of 2-chloro-5-trifluoromethylpyridine based on 3-trifluoromethylpyridine reacted.

### Example 7

5 grams of 3-trifluoromethylpyridine were dissolved in 150 grams of carbon tetrachloride. The solution was heated to reflux and maintained under reflux while chlorine was continuously bubbled through the solution. Alpha, alpha-azobis-isobutyronitrile was added in portions of 100 mg every hour. After 15 hours, analysis by gas-liquid chromatography showed that the major product was 2-chloro-5-trifluoromethylpyridine.

### Example 8

3.2 grams of 3-trifluoromethylpyridine were dissolved in 100 ml of carbon tetrachloride. The solution was saturated with chlorine and heated under reflux while being subjected to irradiation by a mercury-vapour lamp. After a total reaction time of 8 hours analysis by gas-liquid chromatography showed that the major product was 2-chloro-5-trifluoromethylpyridine.

This was confirmed by $^{19}$F nuclear magnetic resonance, which showed that 50% of the 3-trifluoromethylpyridine had reacted and that the product contained 7 moles of 2-chloro-5-trifluoromethylpyridine per mole of 2-chloro-3-trifluoromethylpyridine with very little 2,6-dichloro-3-trifluoromethylpyridine.

## Claims

1. A process for the preparation of 2-chloro-5-trifluoromethylpyridine characterised in that 3-trifluoromethylpyridine is chlorinated:

(i) in the vapour phase optionally in the presence of a diluent at a temperature in the range from 100°C to 500°C, provided that when the temperature is below 250°C the chlorination is carried out in the presence of ultra-violet radiation, or

(ii) in the liquid phase in an organic solvent in the presence of ultra-violet radiation and/or in the presence of a free-radical initiator.

2. A process according to Claim 1 characterised in that the reaction is carried out in the vapour phase at a temperature in the range from 300°C to 450°C.

3. A process according to Claim 2 characterised in that the proportion of chlorine employed is at least 1 mole per mole of 3-trifluoromethylpyridine.

4. A process according to Claim 3 characterised in that the proportion of chlorine employed is from 1 to 6 moles per mole of 3-trifluoromethylpyridine.

5. A process according to any of Claims 2 to 4 characterised in that the reaction mixture contains an organic diluent.

6. A process according to Claim 5 characterised in that the diluent is carbon tetrachloride.

7. A process according to Claim 1 characterised in that the reaction is carried out in the liquid phase at a temperature in the range from 0°C to 100°C.

## Patentansprüche

1. Verfahren zur Herstellung von 2-Chloro-5-trifluoromethylpyridin, dadurch gekennzeichnet, daß 3-Trifluoromethylpyridin

(I) in der Gasphase, gegebenenfalls in Gegenwart eines Verdünnungsmittels, bei einer Temperatur im Bereich von 100° bis 500°C und, für den Fall, daß die Temperatur unter 250°C liegt, in Gegenwart von Ultraviolettstrahlung oder

(II) in der flüssigen Phase in einem organischen Lösungsmittel in Gegenwart von Ultraviolett-

4

strahlung und/oder in Gegenwart eines radikalischen Initiators, chloriert wird.

2. Verfahren nach Anspruch 1, dadurch gekennzeichnet, daß die Reaktion in der Gasphase bei einer Temperatur im Bereich von 300°C bis 450°C ausgeführt wird.

3. Verfahren nach Anspruch 2, dadurch gekennzeichnet, daß die verwendete Menge Chlor mindestens 1 mol je mol 3-Trifluoromethylpyridin beträgt.

4. Verfahren nach Anspruch 3, dadurch gekennzeichnet, daß die verwendete Menge Chlor 1 bis 6 mol je mol 3-Trifluoromethylpyridin beträgt.

5. Verfahren nach einem der Ansprüche 2 bis 4, dadurch gekennzeichnet, daß das Reaktionsgemisch ein organisches Verdünnungsmittel enthält.

6. Verfahren nach Anspruch 4, dadurch gekennzeichnet, daß das Verdünnungsmittel aus Tetrachlorkohlenstoff besteht.

7. Verfahren nach Anspruch 1, dadurch gekennzeichnet, daß die Reaktion in der flüssigen Phase bei einer Temperatur im Bereich von 0 bis 100°C ausgeführt wird.

**Revendications**

1. Procédé de préparation de 2-chloro-5-trifluorométhylpyridine, caractérisé en ce que de la 3-trifluorométhylpyridine est chlorée:

(i) en phase vapeur, éventuellement en présence d'un diluant, à une température comprise dans la plage de 100 à 500°C, sous réserve que lorsque la température est inférieure à 250°C, la chloration soit conduite en présence de rayons ultraviolets, ou

(ii) en phase liquide dans un solvant organique en présence de rayons ultraviolets et/ou en présence d'un initiateur de radicaux libres.

2. Procédé suivant la revendication 1, caractérisé en ce que la réaction est conduite en phase vapeur à une température comprise dans la plage de 300 à 450°C.

3. Procédé suivant la revendication 2, caractérisé en ce que la proportion de chlore utilisée est d'au moins 1 mole par mole de 3-trifluorométhylpyridine.

4. Procédé suivant la revendication 3, caractérisé en ce que la proportion de chlore utilisée est de 1 à 6 moles par mole de 3-trifluorométhylpyridine.

5. Procédé suivant l'une quelconque des revendications 2 à 4, caractérisé en ce que le mélange réactionnel contient un diluant organique.

6. Procédé suivant la revendication 5, caractérisé en ce que la diluant est le tétrachlorure de carbone.

7. Procédé suivant la revendication 1, caractérisé en ce que la réaction est conduite en phase liquide à une température comprise dans la plage de 0 à 100°C.